(19) Europäisches Patentamt
European Patent Office
Office européen des brevets

(11) Numéro de publication: **0 076 221**
**B1**

(12) **FASCICULE DE BREVET EUROPEEN**

(45) Date de publication du fascicule du brevet: **02.05.85**

(51) Int. Cl.[4]: **C 07 C 41/09**, C 07 C 43/23

(21) Numéro de dépôt: **82420131.3**

(22) Date de dépôt: **23.09.82**

(54) **Procédé d'éthérification de phénols.**

(30) Priorité: **29.09.81 FR 8118571**

(43) Date de publication de la demande: **06.04.83 Bulletin 83/14**

(45) Mention de la délivrance du brevet: **02.05.85 Bulletin 85/18**

(84) Etats contractants désignés: **AT BE CH DE GB IT LI LU NL SE**

(56) Documents cités:
**EP - A - 0 037 353**
**GB - A - 2 026 484**

**BULLETIN OF THE CHEMICAL SOCIETY OF JAPAN, vol. 45, no. 11, novembre 1972, pages 3490-3492, (JP);**

(73) Titulaire: **RHONE-POULENC SPECIALITES CHIMIQUES, "Les Miroirs" 18, Avenue d'Alsace, F-92400 Courbevoie (FR)**

(72) Inventeur: **Ratton, Serge, Vaulx Milieu, F-38290 - La Verpilliere (FR)**

(74) Mandataire: **Vignally, Noel et al, RHONE-POULENC RECHERCHES Service Brevets Chimie et Polymères Centre de recherches de Saint-Fons B.P. 62, F-69192 St-Fons Cédex (FR)**

**EP 0 076 221 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un perfectionnement au procédé d'éthérification sélective d'une fonction phénolique de composés pouvant en comporter plusieurs, perfectionnement décrit dans la demande de brevet français N° 80/07628.

Le brevet britannique N° 2026484 décrit un procédé de préparation d'éther phénolique par réaction d'un phénol ou de l'un de ses dérivés avec un ester de l'acide carbonique en présence d'un système catalytique comprenant une base et un iodure organique ou inorganique.

Lorsqu'il est appliqué à un polyphénol, ce procédé ne permet pas d'obtenir sélectivement le monoéther correspondant.

Le «Bulletin of the Chemical Society of Japan», volume 45/1972 (pp. 3490-3492), décrit un procédé d'O-alkylation de monophénols, éventuellement substitués, dont la fonction phénolique est sous forme de phénolate de sodium ou de potassium. Un tel procédé n'est pas présenté comme étant applicable à la monoéthérification sélective des polyphénols.

Le présent perfectionnement consiste à associer, au sel d'acide carboxylique utilisé dans le procédé de base, une quantité efficace d'halogénure métallique.

L'éthérification est dite sélective parce que, au cours de la mise en œuvre du procédé selon l'invention, une seule fonction phénolique est éthérifiée, lorsque ledit composé phénolique en comporte plusieurs sur le même cycle aromatique.

Plus précisément, l'invention a pour objet un nouveau procédé d'éthérification d'une fonction phénolique d'un composé de formule générale:

$$HO - Ar - (R)_n (I)$$

dans laquelle:

— Ar représente un radical aromatique constitué par un cycle benzénique ou par une structure formée par plusieurs cycles benzéniques orthocondensés ou ortho- et péricondensés,

— les substituants R, identiques ou différents, représentent un groupement hydroxyle, un radical alkyle linéaire ou ramifié ayant 1 à 6 atomes de carbone, un radical alkényle linéaire ou ramifié ayant 2 à 6 atomes de carbone, un radical phényle, un radical cycloalkyle, un radical phénylalkyle dans lequel la chaîne aliphatique comporte 1 à 4 atomes de carbone, un radical cycloalkylalkyle dans lequel la chaîne aliphatique comporte 1 à 4 atomes de carbone, un atome d'halogène, un groupement nitro, un groupement amine, un groupement aldéhyde $-CHO$, ou un groupement nitrile,

— n est un nombre de 0 à 5,

par réaction avec un agent d'éthérification choisi dans le groupe constitué par les carboxylates d'alkyle, le radical alkyle linéaire ou ramifié ayant 1 à 6 atomes de carbone, les carboxylates d'alkényle, le radical alkényle linéaire ou ramifié ayant 3 à 6 atomes de carbone et les composés susceptibles de former de tels carboxylates, caractérisé en ce que l'on opère en présence de quantités efficaces d'un sel d'acide carboxylique choisi parmi les carboxylates d'un métal alcalin, d'ammonium ou d'un métal alcalino-terreux et d'un halogénure métallique choisi parmi les iodures d'un métal alcalin, les iodures d'un métal alcalino-terreux ou l'iodure d'ammonium.

L'invention peut également être présentée comme un procédé de préparation de monoéther de phénol par réaction d'un composé de formule générale (I) avec un agent d'éthérification choisi dans le groupe constitué par les carboxylates d'alkyle, le radical alkyle linéaire ou ramifié ayant 1 à 6 atomes de carbone, les carboxylates d'alkényle, le radical alkényle linéaire ou ramifié ayant 3 à 6 atomes de carbone et les composés susceptibles de former de tels carboxylates, ledit procédé se caractérisant en ce que l'on opère en présence de quantités efficaces d'un sel d'acide carboxylique tel que ceux indiqués précédemment et d'un halogénure métallique tel que ceux indiqués précédemment.

De manière préférée, on applique le procédé selon l'invention à des composés phénoliques de formule (I) dans laquelle:

— le radical Ar correspond au benzène, au naphtalène, à l'anthracène ou au phénanthrène,

— les substituants R, identiques ou différents, représentent un groupement hydroxyle, un radical alkyle linéaire ou ramifié ayant 1 à 4 atomes de carbone tel que méthyle, éthyle, isopropyle, n-propyle, n-butyle, isobutyle ou tertiobutyle, un radical alkényle linéaire ou ramifié ayant 2 à 4 atomes de carbone tel que vinyle, allyle, propène-1 yle, isopropényle, butène-1 yle, butène-2 yle, butène-3 yle, méthyl-1 propène-1 yle, méthyl-1 propène-2 yle, méthyl-2 propène-1 yle, méthyl-2 propène-2 yle, un radical phényle, un radical cyclohexyle, un radical phénylalkyle dans lequel la chaîne aliphatique comporte 1 à 3 atomes de carbone tel que benzyle, phénéthyle, phénylpropyle et phénylisopropyle, un radical cyclohexylalkyle dans lequel la chaîne aliphatique comporte 1 à 3 atomes de carbone, un atome de chlore, un atome de brome ou un groupement nitro,

— n est un nombre entier de 0 à 3.

A titre d'exemples de tels composés phénoliques, on peut citer:

— les monophénols comme le phénol, le naphtol-1, le naphtol-2, le phénanthrol-1, le phénanthrol-2, le phénanthrol-3, le phénanthrol-9, l'anthrol-1, l'anthrol-2, l'anthrol-9,

— les diphénols comme le résorcinol, le pyrocatéchol, l'hydroquinone, le dihydroxy-1,2 naphtalène, le dihydroxy-1,3 naphtalène, le dihydroxy-1,4 naphtalène, le dihydroxy-1,5 naphtalène, le dihydroxy-1,6 naphtalène, le dihydroxy-1,7 naphtalène, le dihydroxy-1,8 naphtalène, le dihydroxy-2,3 naphtalène, le dihydroxy-2,6 naphtalène, le dihydroxy-2,7 naphtalène, le dihydroxy-1,2 anthracène, le dihydroxy-1,5 anthracène, le dihydroxy-1,8 anthracène, le dihydroxy-2,6 anthracène, le dihydroxy-9,10 anthracène, le dihydroxy-3,4 phénanthrène,

— les triphénols comme le pyrogallol, le trihydroxy-1,2,4 benzène, le trihydroxy-1,3,5 benzène, le trihydroxy-1,2,9 anthracène, le trihydroxy-1,2,10 anthracène, le trihydroxy-1,4,9 anthracène, le trihydroxy-1,5,9 anthracène, le trihydroxy-1,8,9 anthracène, le trihydroxy-2,3,9 anthracène, le trihydroxy-3,4,5 phénanthrène,

— les mono- ou diphénols portant également un ou plusieurs autres substituants R comme: le chloro-2 phénol, le chloro-3 phénol, le chloro-4 phénol, le bromo-2 phénol, le bromo-3 phénol, le bromo-4 phénol, le nitro-2 phénol, le nitro-3 phénol, le nitro-4 phénol, le méthyl-2 phénol, le méthyl-3 phénol, le méthyl-4 phénol, l'éthyl-2 phénol, l'éthyl-3 phénol, l'éthyl-4 phénol, l'isopropyl-2 phénol, l'isopropyl-3 phénol, l'isopropyl-4 phénol, le propyl-2 phénol, le propyl-3 phénol, le propyl-4 phénol, le (propène-1 yle)-4 phénol, l'allyl-2 phénol, l'allyl-4 phénol, le butyl-3 phénol, le butyl-4 phénol, l'isobutyl-4 phénol, le tertiobutyl-2 phénol, le tertiobutyl-3 phénol, le tertiobutyl-4 phénol, le benzyl-2 phénol, le benzyl-4 phénol, le cyclohexyl-2 phénol, le dichloro-2,3 phénol, le dichloro-2,4 phénol, le dichloro-2,5 phénol, le dichloro-2,6 phénol, le dichloro-3,4 phénol, le dichloro-3,5 phénol, le diméthyl-1,2 hydroxy-3 benzène, le diméthyl-1,2 hydroxy-4 benzène, le diméthyl-1,3 hydroxy-5 benzène, le diméthyl-1,3 hydroxy-2 benzène, le diméthyl-1,4 hydroxy-2 benzène, le diméthyl-2,4 hydroxy-1 benzène, le tertiobutyl-1 hydroxy-2 méthyl-4 benzène, le tertiobutyl-2 hydroxy-1 méthyl-4 benzène, le tertiobutyl-2 éthyl-4 hydroxy-1 benzène, le tertiobutyl-4 éthyl-2 hydroxy-1 benzène, le ditertiobutyl-1,3 hydroxy-2 benzène, le ditertiobutyl-2,4 hydroxy-1 benzène, l'hydroxy-2 isopropyl-4 méthyl-1 benzène, l'allyl-2 chloro-4 hydroxy-1 benzène, l'hydroxy-1 triméthyl-2,4,5 benzène, l'hydroxy-2 triméthyl-1,3,5 benzène, l'hydroxy-2 tritertiobutyl-1,3,5 benzène, le ditertiobutyl-1,3 hydroxy-2 méthyl-5 benzène, le ditertiobutyl-1,5 hydroxy-2 méthyl-3 benzène, le ditertiobutyl-1,5 hydroxy-2 méthyl-4 benzène, le tertiobutyl-1 diméthyl-2,5 hydroxy-4 benzène, le tertiobutyl-1 diméthyl-3,5 hydroxy-2 benzène, le tertiobutyl-1 diméthyl-4,5 hydroxy-2 benzène, le tertiobutyl-5 diméthyl-1,3 hydroxy-2 benzène, le chloro-1 diméthyl-2,3 hydroxy-4 benzène, le chloro-1 diméthyl-2,3 hydroxy-5 benzène, le chloro-1 diméthyl-2,4 hydroxy-5 benzène, le chloro-1 diméthyl-2,5 hydroxy-4 benzène, le chloro-1 diméthyl-3,4 hydroxy-2 benzène, le chloro-1 diméthyl-4,5 hydroxy-2 benzène, le chloro-2 diméthyl-1,3 hydroxy-5 benzène, le chloro-2 diméthyl-1,5 hydroxy-3 benzène, le chloro-2 diméthyl-3,4 hydroxy-1 benzène, le chloro-5 diméthyl-1,3 hydroxy-2 benzène, le diméthyl-1,2 hydroxy-3 nitro-5 benzène, le diméthyl-1,2 hydroxy-4 nitro-5 benzène, le diméthyl-1,3 hydroxy-2 nitro-4 benzène, le diméthyl-1,3 hydroxy-2 nitro-5 benzène, le diméthyl-1,4 hydroxy-2 nitro-3 benzène, le diméthyl-1,4 hydroxy-2 nitro-5 benzène, le diméthyl-1,5 hydroxy-2 nitro-3 benzène, le diméthyl-1,5 hydroxy-3 nitro-2 benzène, le diméthyl-2,5 hydroxy-1 nitro-3 benzène, le nitro-8 naphtol-1, le nitro-1 naphtol-2, le nitro-5 naphtol-2, le méthyl-1 naphtol-2, le bromo-1 dihydroxy-2,4 benzène, le bromo-1 dihydroxy-3,5 benzène, le bromo-2 dihydroxy-1,3 benzène, le bromo-2 dihydroxy-1,4 benzène, le bromo-4 dihydroxy-1,2 benzène, le butyl-1 dihydroxy-2,4 benzène, le chloro-1 dihydroxy-2,3 benzène, le chloro-1 dihydroxy-2,4 benzène, le chloro-1 dihydroxy-3,5 benzène, le chloro-2 dihydroxy-1,3 benzène, le chloro-2 dihydroxy-1,4 benzène, le chloro-4 dihydroxy-1,2 benzène, le dihydroxy-2,4 éthyl-1 benzène, le dihydroxy-2,4 isobutyl-1 benzène, le dihydroxy-1,2 isopropyl-4 benzène, le dihydroxy-1,4 isopropyl-2 benzène, le dihydroxy-2,4 isopropyl-1 benzène, le dihydroxy-2,3 isopropyl-1 méthyl-4 benzène, le dihydroxy-1,4 isopropyl-2 méthyl-5 benzène, le dihydroxy-1,2 méthyl-3 benzène, le dihydroxy-1,3 méthyl-2 benzène, le dihydroxy-1,3 nitro-2 benzène, le dihydroxy-1,4 nitro-2 benzène, le dihydroxy-1,2 propyl-4 benzène, le dihydroxy-1,3 propyl-5 benzène, le dihydroxy-2,4 propyl-1 benzène, le dichloro-1,2 dihydroxy-4,5 benzène, le dichloro-1,3 dihydroxy-2,5 benzène, le dichloro-1,4 dihydroxy-2,5 benzène, le dichloro-1,5 dihydroxy-2,3 benzène, le dichloro-1,5 dihydroxy-2,4 benzène, le dichloro-2,3 dihydroxy-1,4 benzène, le dihydroxy-1,2 diméthyl-3,5 benzène, le dihydroxy-1,2 diméthyl-4,5 benzène, le dihydroxy-1,3 diméthyl-2,4 benzène, le dihydroxy-1,3 diméthyl-2,5 benzène, le dihydroxy-1,4 diméthyl-2,3 benzène, le dihydroxy-1,4 diméthyl-2,5 benzène, le dihydroxy-1,5 diméthyl-2,4 benzène, le dihydroxy-1,5 diméthyl-3,4 benzène, le dihydroxy-2,5 diméthyl-1,3 benzène, le dihydroxy-1,3 dinitro-2,4 benzène.

Le procédé selon l'invention est plus particulièrement appliqué au phénol, au naphtol-1, au naphtol-2, au méthyl-2 phénol, au méthyl-3 phénol, au méthyl-4 phénol, aux monochlorophénols, aux dichlorophénols, aux monoéthylphénols, au pyrocatéchol, au résorcinol, à l'hydroquinone, au dihydroxy-1,2 naphtalène, au dihydroxy-1,3 naphtalène, au dihydroxy-1,4 naphtalène, au dihydroxy-1,5 naphtalène, au dihydroxy-1,6 naphtalène, au dihydroxy-1,7 naphtalène, au dihydroxy-1,8 naphtalène, au dihydroxy-2,3 naphtalène, au dihydroxy-2,6 naphtalène, au dihydroxy-2,7 naphtalène.

La concentration du composé phénolique de formule (I) dans le milieu réactionnel n'est pas critique. Elle varie très largement, notamment en fonction de la solubilité de ce composé dans ledit milieu, qui est constitué par ledit composé phénolique, l'agent d'éthérification, le sel d'acide carboxylique, l'halogénure métallique et, le cas échéant, par d'autres éléments qui seront détaillés plus loin et qui peuvent consister en particulier en des adjuvants non indispensables, mais favorables à la mise en œuvre du procédé selon l'invention et/ou en un tiers solvant.

On peut également réaliser l'éthérification du composé phénolique en masse, ce composé se trouvant à l'état liquide. Généralement, le composé phénolique représente en poids de 1 à 90% du poids du milieu réactionnel.

Parmi les agents d'éthérification que l'on peut mettre en œuvre dans le procédé selon l'invention, on peut citer notamment les esters:

— des acides carboxyliques aliphatiques saturés ou insaturés monofonctionnels comportant notamment 2 à 18 atomes de carbone ou polyfonctionnels comportant notamment 3 à 18 atomes de carbone,

— des acides aromatiques mono- ou polyfonctionnels,

— des acides arylaliphatiques mono- ou polyfonctionnels, ou

— des acides cycloaliphatiques mono- ou polyfonctionnels avec des alcools tels que le méthanol, l'éthanol, le n-propanol, l'isopropanol, le butanol-1, le butanol-2, le tertiobutanol, le propène-2 ol-1, le méthyl-2 propène-2 ol-1, le butène-2 ol-1, le butène-3 ol-1, le butène-3 ol-2.

On peut également mettre en œuvre des composés susceptibles de former de tels esters *in situ* et notamment un mélange d'un alcool tel que ceux énumérés ci-avant avec un acide carboxylique tel que ceux définis précédemment. Cette variante est celle qui est préférée notamment pour des raisons de commodité, ces réactifs étant parfois plus facilement disponibles que les esters eux-mêmes. En outre, il n'est pas nécessaire d'avoir des quantités stœchiométriques d'alcool et d'acide carboxylique. Enfin, la réaction d'éthérification est en général plus rapide avec ces réactifs qu'avec les esters. Le rapport molaire alcool/acide carboxylique peut varier dans de larges limites, par exemple entre 0,02 et 50, plus fréquemment entre 0,1 et 40. Le rapport molaire acide carboxylique/composé phénolique peut également varier très largement. Généralement, il se situe entre 0,01 et 100. De préférence, ce rapport est compris entre 0,05 et 20.

Par la suite, lorsqu'il sera question d'agent d'éthérification, ce terme englobera aussi bien l'ester lui-même que les mélanges dans les proportions définies précédemment de l'alcool et de l'acide carboxylique correspondants.

La quantité d'agent d'éthérification utilisée est généralement choisie de telle façon que le rapport molaire ester et/ou alcool entrant dans la composition dudit agent éthérifiant sur composé phénolique de formule (I) est égal ou supérieur à 0,3 et de préférence égal ou supérieur à 0,5.

On peut citer à titre d'illustration des acides carboxyliques précédents des monoacides aliphatiques saturés comme l'acide acétique, les acides propanoïque, n-butanoïque, méthyl-2 propanoïque, n-pentanoïque, méthyl-2 butanoïque, méthyl-3 butanoïque, diméthyl-3,3 butanoïque, n-hexanoïque, méthyl-2 pentanoïque, méthyl-3 pentanoïque, méthyl-4 pentanoïque; des monoacides aliphatiques insaturés comme les acides propénoïque, butène-2 oïque, méthyl-2 propénoïque, butène-3 oïque, méthyl-2 butène-2 oïque cis (acide angélique), méthyl-2 butène-2 oïque trans (acide tiglique), pentène-4 oïque, hexène-3 oïque, hexène-4 oïque; des diacides aliphatiques saturés comme l'acide malonique, l'acide succinique, l'acide glutarique, l'acide adipique; des diacides éthyléniques comme l'acide maléique et l'acide fumarique; des mono- ou des diacides aromatiques comme l'acide benzoïque, l'acide orthophtalique, l'acide isophtalique, l'acide téréphtalique, les acides mononitrobenzoïques, les acides monochlorobenzoïques; des acides arylaliphatiques comme les acides phénylacétique, phényl-2 propanoïque, phényl-4 propanoïque; des acides cycloaliphatiques comme l'acide cyclohexanedicarboxylique-1,3, l'acide cyclohexanedicarboxylique-1,4.

On peut également utiliser des mélanges comprenant un ester d'acide carboxylique et l'alcool libre correspondant ou un ester d'acide carboxylique avec l'alcool libre et l'acide carboxylique libre correspondant à cet ester. Dans le présent texte, lorsque l'on parle d'agent d'éthérification, il est entendu qu'il peut s'agir d'un tel mélange, sans qu'il soit nécessaire de le préciser chaque fois.

Les agents d'éthérification que l'on préfère utiliser généralement sont les esters du méthanol, de l'éthanol, du n-propanol, du propène-2 ol-1 et du méthyl-2 propène-2 ol-1 et d'un acide carboxylique, ou les mélanges tels que définis précédemment de l'un de ces alcools avec un acide carboxylique. On choisit fréquemment l'acide carboxylique parmi les acides aliphatiques saturés mono- ou difonctionnels ayant 2 à 6 atomes de carbone, l'acide benzoïque et les acides ortho-, méta- ou téréphtaliques. Parmi ces agents d'éthérification, on préfère le plus souvent utiliser les acétates, les propionates et les succinates ou les mélanges constitués par l'acide acétique ou l'acide propionique ou l'acide succinique avec l'un des alcools cités ci-avant.

Enfin, parmi ces agents d'éthérification préférés, on préfère plus particulièrement employer les esters du méthanol et les esters de l'éthanol, tout spécialement leurs acétates ou les mélanges constitués par le méthanol et l'un de ces acides carboxyliques ou l'éthanol et l'un de ces acides carboxyliques et tout spécialement les mélanges constitués par l'un de ces deux alcools avec l'acide acétique.

L'agent d'éthérification peut constituer le milieu solvant dans lequel s'opère la réaction d'éthérification selon le procédé de l'invention. Mais il est bien entendu que l'on peut également utiliser un tiers solvant, liquide dans les conditions de mise en œuvre du procédé, dans la mesure où ledit tiers solvant est inerte vis-à-vis des réactifs et stable aux températures auxquelles est effectuée la réaction.

L'eau peut servir de tiers solvant. Mais sa présence procure un avantage particulier et joue un rôle remarquable, distinct du simple rôle de tiers solvant. En effet, la présence d'eau conduit à une augmentation du rendement en monoéther du composé phénolique et à une diminution corrélative des réactions parasites.

Lorsque l'on opère en présence d'eau, celle-ci peut représenter de 1 à 95% en volume du milieu solvant liquide. De préférence, le milieu solvant liquide comprend de 20 à 80% en volume d'eau.

Le sel d'acide carboxylique servant de catalyseur dans le procédé selon l'invention peut être tout

carboxylate, notamment les carboxylates des métaux alcalins, d'ammonium et des métaux alcalino-terreux. A titre d'exemples, on peut citer notamment les carboxylates de sodium, de potassium, de lithium et d'ammonium; on peut également citer les carboxylates de calcium, de magnésium et de baryum.

Les acides carboxyliques servant à obtenir de tels carboxylates peuvent être les acides mono- ou polyfonctionnels, aliphatiques saturés ou insaturés, aromatiques, arylaliphatiques, cycloaliphatiques dont les cycles peuvent être substitués par un ou plusieurs radicaux.

Ces acides peuvent être par exemple ceux qui ont été cités précédemment lors de la définition des esters servant d'agents d'éthérification.

Parmi tous les carboxylates que l'on peut utiliser, on préfère généralement les sels des acides carboxyliques aliphatiques saturés, monofonctionnels ayant 2 à 6 atomes de carbone, tels que notamment l'acide acétique, les acides propanoïque, n-butanoïque, n-pentanoïque, n-hexanoïque, méthyl-2 propanoïque, méthyl-2 butanoïque, méthyl-3 butanoïque, diméthyl-3,3 butanoïque, méthyl-2 pentanoïque, méthyl-3 pentanoïque et méthyl-4 pentanoïque, ou difonctionnels ayant 3 à 6 atomes de carbone, tels que notamment l'acide malonique, l'acide succinique, l'acide glutarique et l'acide adipique, les sels de l'acide benzoïque et ceux des acides orthophtalique, isophtalique et téréphtalique. Parmi les sels de ces acides, on préfère les sels de métaux alcalins.

De manière encore préférée, on emploie les sels de sodium ou de potassium de ces acides. Parmi ces derniers sels, on utilise préférentiellement l'acétate de sodium, le propionate de sodium et le succinate de sodium.

Il est commode de choisir l'agent d'éthérification et le sel d'acide carboxylique servant de catalyseur de telle façon qu'ils proviennent (ou contiennent) du même acide carboxylique, mais cela n'est pas indispensable.

La quantité de sel d'acide carboxylique présent dans le milieu peut varier dans de larges limites. Si on exprime cette quantité par rapport au composé phénolique, elle n'est généralement pas inférieure à 0,01 fois le poids dudit composé phénolique. La quantité maximale n'est pas critique. Habituellement, elle ne dépasse pas 50 fois le poids du composé phénolique. On préfère le plus souvent utiliser des rapports pondéraux sel d'acide carboxylique/composé phénolique variant de 0,05 à 20.

Une variante intéressante consiste à mettre en œuvre un diacide carboxylique dont une fonction acide est salifiée, en général par un métal alcalin, et dont l'autre fonction est soit libre, soit estérifiée par un alcool tel que ceux précédemment cités, le plus souvent le méthanol ou l'éthanol.

Comme halogénure métallique, on peut utiliser par exemple un iodure métallique. Ce sera généralement un iodure de métal alcalin comme les iodures de sodium, de potassium ou de lithium, un iodure de métal alcalino-terreux comme les iodures de calcium, de magnésium ou de baryum ou l'iodure d'ammonium.

Parmi ces iodures, on préfère plus particulièrement les iodures de sodium et de potassium.

L'halogénure métallique et le sel d'acide carboxylique servant de catalyseur peuvent correspondre au même cation alcalin ou à des cations alcalins différents.

Les quantités d'halogénure métallique qui sont utilisées peuvent varier dans de très larges limites.

Si l'on exprime cette quantité en poids par rapport au composé phénolique, elle se situe généralement entre 0,01 et 50 fois le poids dudit composé phénolique.

On préfère le plus souvent utiliser des rapports pondéraux halogénure métallique/composé phénolique compris entre 0,05 et 20.

Le procédé selon l'invention nécessite pour sa mise en œuvre un chauffage des réactifs; la température à laquelle on opère peut varier de 150 à 350° C. Elle se situe de préférence entre 200 et 300° C.

La pression n'est pas un paramètre critique de la réaction. Habituellement, elle est constituée par la pression autogène obtenue par chauffage, à la température désirée, du mélange réactionnel dans un appareillage adéquat fermé. Elle se situe généralement entre 10 et 100 bar. Mais elle peut atteindre des valeurs plus élevées, car il est possible, sans sortir du cadre de l'invention, de créer dans l'appareil utilisé pour la réaction une pression initiale à froid supérieure à la pression atmosphérique, par exemple au moyen d'un gaz inerte tel que l'azote.

L'appareillage utilisé n'est pas spécifique au procédé de l'invention. Simplement, il doit présenter certaines caractéristiques: il doit pouvoir résister aux pressions que l'on atteint lors du chauffage, il doit être étanche et évidemment ne pas être attaqué par les réactifs utilisés.

Pratiquement, le procédé selon l'invention peut être mis en œuvre de la manière suivante: on charge les différents constituants du mélange réactionnel, tels que définis précédemment, dans l'appareillage adéquat. On chauffe à la température désirée, de préférence sous agitation mais sans que cela soit véritablement indispensable, pendant une durée pouvant varier de quelques minutes à plus de 20 h par exemple. Cependant, cette durée est généralement de l'ordre de quelques heures, par exemple de 2 à 10 h selon la température à laquelle on opère.

En fin de réaction, l'appareil est refroidi et la masse réactionnelle finale est traitée de manière classique, selon les réactifs utilisés; si le milieu contient de l'eau, les composés organiques autres que le sel d'acide carboxylique sont extraits par un solvant non miscible à l'eau. Si le milieu ne contient pas ou très peu d'eau, on peut généralement séparer le sel d'acide carboxylique et l'halogénure métallique par filtration soit directement, soit après les avoir précipités par addition d'un solvant organique dans lequel ils ne sont pas solubles, mais qui dissout les composés formés lors de la réaction. On peut aussi rajouter de l'eau

au milieu avant de procéder à l'extraction des composés organiques.

Les produits obtenus sont séparés, notamment du composé phénolique n'ayant pas été transformé, par des opérations courantes dans le domaine de la chimie, puis dosés si nécessaire, également par des méthodes bien connues de l'homme du métier.

Un procédé de séparation des éthers formés au cours de la réaction consiste notamment à les extraire à l'aide d'un solvant approprié non miscible à l'eau. On peut utiliser par exemple le cyclohexane comme solvant d'extraction. De préférence, on opère à une température comprise entre 50° C et le point d'ébullition du mélange réactionnel final. En général, cette température, non critique, est de l'ordre de 60° C. On extrait ainsi la grande majorité des produits d'éthérification formés, tandis que le phénol non transformé reste pour l'essentiel dans le milieu réactionnel avec le sel d'acide carboxylique, l'halogénure métallique et l'acide carboxylique libre. La masse réactionnelle finale obtenue après l'extraction peut ainsi être recyclée après un éventuel rajout d'agent d'éthérification.

L'utilisation d'halogénure métallique en association avec un sel d'acide carboxylique permet d'augmenter la cinétique de la réaction d'éthérification des composés phénoliques.

On peut ainsi, tout en obtenant un taux de transformation du composé phénolique du même ordre de grandeur que celui que l'on a avec le procédé utilisant le seul sel d'acide carboxylique, soit diminuer la durée de la réaction, soit opérer à une température plus basse. Le procédé selon l'invention représente donc une amélioration du procédé initial selon la demande N° 80/07628, en procurant une meilleure productivité industrielle.

Les monoéthers obtenus par le procédé selon l'invention peuvent soit être utilisés directement, soit servir d'intermédiaires pour la synthèse de composés plus complexes.

Le méthoxy-2 phénol (ou gaïacol), par exemple, est utilisé dans l'industrie pharmaceutique; il sert également d'intermédiaire pour la préparation de la vanilline.

Dans l'exemple et l'essai comparatif qui vont suivre, les dosages, sauf mention contraire, sont effectués par chromatographie gaz/liquide.

*Exemple*

Dans un tube de verre résistant à la pression, on introduit les réactifs suivants:

- Acétate de sodium anhydre: 0,77 g
- Iodure de potassium: 0,77 g
- Acide acétique: 0,26 g
- Eau distillée: 3,4 ml
- Méthanol: 3,4 ml
- Pyrocatéchol: 1,56 g

Le tube est scellé, puis on chauffe à 250° C sous agitation et on maintient à cette température pendant 2 h.

En fin d'essai, on refroidit et on extrait du mélange aqueux le pyrocatéchol non transformé et le gaïacol formé, à l'aide d'éther isopropylique. On dose les produits par chromatographie gaz/liquide. On trouve:

- Taux de transformation (TT) du pyrocatéchol: 30,0%
- Rendement en gaïacol par rapport au pyrocatéchol transformé (RT): 97%
- Rendement en vératrole (RT): 3%

*Essai comparatif selon le procédé de base*

Dans un tube de verre résistant à la pression, on introduit les réactifs suivants:

- Acétate de sodium anhydre: 1,54 g
- Acide acétique: 0,26 g
- Eau distillée: 3,4 ml
- Méthanol: 3,4 ml
- Pyrocatéchol: 1,56 g

Le tube est scellé, puis on chauffe à 250° C sous agitation et on maintient à cette température pendant 2 h.

En fin d'essai, on refroidit et on extrait du mélange aqueux le pyrocatéchol non transformé et le gaïacol formé, à l'aide d'éther isopropylique. On dose les produits par chromatographie gaz/liquide. On trouve:

- Taux de transformation (TT) du pyrocatéchol: 18,0%
- Rendement en gaïacol par rapport au pyrocatéchol transformé (RT): 97%
- Rendement en vératrole (RT): 3%

## Revendications

1. Procédé d'éthérification d'une fonction phénolique de composé de formule générale (I):

$$HO - Ar - (R)_n (I)$$

dans laquelle:

— Ar représente un radical aromatique constitué par un cycle benzénique ou par plusieurs cycles benzéniques orthocondensés ou ortho- et péricondensés,

— les substituants R, identiques ou différents, représentent un groupement hydroxyle, un radical alkyle linéaire ou ramifié ayant 1 à 6 atomes de carbone, un radical alkényle linéaire ou ramifié ayant 2 à 6 atomes de carbone, un radical phényle, un radical cycloalkyle, un radical phénylalkyle dans lequel la chaîne aliphatique comporte 1 à 4 atomes de carbone, un radical cycloalkylalkyle dans lequel la chaîne aliphatique comporte 1 à 4 atomes de carbone, un atome d'halogène, un groupement nitro, un groupement amine, un groupement aldéhyde, un groupement nitrile,

— n est un nombre de 0 à 5,

par réaction avec un agent d'éthérification choisi dans le groupe constitué par les carboxylates d'alkyle, le radical alkyle linéaire ou ramifié ayant 1 à 6 atomes de carbone, les carboxylates d'alkényle, le radical alkényle linéaire ou ramifié ayant 3 à 6 atomes de carbone et les composés susceptibles de former de tels carboxylates, caractérisé en ce que l'on opère en présence de quantités efficaces d'un sel d'acide carboxylique choisi parmi les

carboxylates d'un métal alcalin, d'ammonium ou d'un métal alcalino-terreux et d'un halogénure métallique choisi parmi les iodures d'un métal alcalin, les iodures d'un métal alcalino-terreux ou l'iodure d'ammonium.

2. Procédé selon la revendication 1, caractérisé en ce qu'il est appliqué à un composé de formule (I) dans laquelle:

– le radical Ar correspond au benzène, au naphtalène, à l'anthracène ou au phénanthrène,

– les substituants R, identiques ou différents, représentent un groupement hydroxyle, un radical alkyle linéaire ou ramifié ayant 1 à 4 atomes de carbone, un radical alkényle linéaire ou ramifié ayant 2 à 4 atomes de carbone, un radical phényle, un radical cyclohexyle, un radical phénylalkyle dans lequel la chaîne aliphatique comporte 1 à 3 atomes de carbone, un radical cyclohexylalkyle dans lequel la chaîne aliphatique comporte 1 à 3 atomes de carbone, un atome de chlore, un atome de brome ou un groupement nitro,

– n est un nombre entier de 0 à 3.

3. Procédé selon l'une des revendications 1 ou 2, caractérisé en ce qu'il est appliqué au phénol, au naphtol-1, au naphtol-2, au méthyl-2 phénol, au méthyl-3 phénol, au méthyl-4 phénol, aux monochlorophénols, aux dichlorophénols, aux monoéthylphénols, aux mononitrophénols, au pyrocatéchol, au résorcinol, à l'hydroquinone, au dihydroxy-1,2 naphtalène, au dihydroxy-1,3 naphtalène, au dihydroxy-1,4 naphtalène, au dihydroxy-1,5 naphtalène, au dihydroxy-1,6 naphtalène, au dihydroxy-1,7 naphtalène, au dihydroxy-1,8 naphtalène, au dihydroxy-2,3 naphtalène, au dihydroxy-2,6 naphtalène, au dihydroxy-2,7 naphtalène.

4. Procédé selon l'une des revendications 1 à 3, caractérisé en ce que l'agent d'éthérification est choisi parmi les esters:

– des acides carboxyliques aliphatiques saturés ou insaturés monofonctionnels comportant notamment 2 à 18 atomes de carbone ou polyfonctionnels comportant notamment 3 à 18 atomes de carbone,

– des acides aromatiques mono- ou polyfonctionnels,

– des acides arylaliphatiques mono- ou polyfonctionnels, ou

– des acides cycloaliphatiques mono- ou polyfonctionnels avec des alcools tels que le méthanol, l'éthanol, le n-propanol, l'isopropanol, le butanol-1, le butanol-2, le tertiobutanol, le propène-2 ol-1, le méthyl-2 propène-2 ol-1, le butène-2 ol-1, le butène-3 ol-1, le butène-3 ol-2.

5. Procédé selon l'une des revendications 1 à 3, caractérisé en ce que l'agent d'éthérification est constitué par un mélange:

– d'un alcool tel que le méthanol, l'éthanol, le n-propanol, l'isopropanol, le butanol-1, le butanol-2, le tertiobutanol, le propène-2 ol-1, le méthyl-2 propène-2 ol-1, le butène-2 ol-1, le butène-3 ol-1, le butène-3 ol-2, et

– d'un acide carboxylique tel que:

– un acide carboxylique aliphatique saturé ou insaturé monofonctionnel comportant notamment 2 à 18 atomes de carbone ou polyfonctionnel comportant notamment 3 à 18 atomes de carbone,

– un acide aromatique mono- ou polyfonctionnel,

– un acide arylaliphatique mono- ou polyfonctionnel, ou

– un acide cycloaliphatique mono- ou polyfonctionnel.

6. Procédé selon la revendication 5, caractérisé en ce que le rapport molaire alcool/acide carboxylique est compris entre 0,02 et 50 et de préférence entre 0,1 et 40.

7. Procédé selon l'une des revendications 4 à 6, caractérisé en ce que l'acide carboxylique entrant dans la composition de l'agent d'éthérification est un acide aliphatique saturé mono- ou difonctionnel ayant de 2 à 6 atomes de carbone, l'acide benzoïque ou un des acides ortho-, méta- et téréphtaliques.

8. Procédé selon l'une des revendications 1 à 7, caractérisé en ce que le sel d'acide carboxylique utilisé est un carboxylate de métal alcalin.

9. Procédé selon l'une des revendications 1 à 8, caractérisé en ce que le sel d'acide carboxylique utilisé est un sel d'un acide aliphatique saturé mono- ou difonctionnel ayant 2 à 6 atomes de carbone, de l'acide benzoïque ou de l'un des acides ortho-, méta- et téréphtaliques.

10. Procédé selon l'une des revendications 1 à 9, caractérisé en ce que le rapport pondéral sel d'acide carboxylique/composé phénolique est compris entre 0,01 et 50 et de préférence entre 0,05 et 20.

11. Procédé selon l'une des revendications 1 à 10, caractérisé en ce que l'halogénure métallique est l'iodure de sodium ou de potassium.

12. Procédé selon l'une des revendications 1 à 11, caractérisé en ce que le rapport pondéral halogénure métallique/composé phénolique est compris entre 0,01 et 50 et de préférence entre 0,05 à 20.

13. Procédé selon l'une des revendications 1 à 12, caractérisé en ce que l'on opère en présence d'eau.

14. Procédé selon la revendication 13, caractérisé en ce que l'eau représente de 1 à 95% en volume du milieu solvant liquide et de préférence de 20 à 80%.

15. Procédé selon l'une des revendications 1 à 3 et 5 et 6, caractérisé en ce que l'on utilise un diacide carboxylique dont une fonction acide est libre et l'autre est salifiée.

16. Procédé selon l'une des revendications 1 à 15, caractérisé en ce que l'on opère à une température située entre 150 et 350° C, et de préférence entre 200 et 300° C.

17. Procédé selon l'une des revendications 1 à 16, caractérisé en ce que l'on utilise un rapport molaire ester ou alcool entrant dans la composition de l'agent d'éthérification/composé phénolique égal ou supérieur à 0,3, et de préférence égal ou supérieur à 0,5.

18. Procédé selon l'une des revendications 1 à 17, caractérisé en ce que les éthers formés se

trouvant dans la masse réactionnelle finale sont extraits par le cyclohexane.

## Patentansprüche

1. Verfahren zum Veräthern einer Phenolfunktion der Verbindung der allgemeinen Formel (I):

$$HO - Ar - (R)_n (I)$$

in welcher:

– Ar einen aromatischen Rest, bestehend aus einem Benzolring oder aus mehreren orthokondensierten oder ortho- und perikondensierten Benzolringen, darstellt,

– die Substituenten R unabhängig voneinander eine Hydroxylgruppe, einen geradkettigen oder verzweigten Alkylrest mit 1 bis 6 Kohlenstoffatomen, einen geradkettigen oder verzweigten Alkenylrest mit 2 bis 6 Kohlenstoffatomen, einen Phenylrest, einen Cycloalkylrest, einen Phenylalkylrest, worin die aliphatische Kette 1 bis 4 Kohlenstoffatome enthält, einen Cycloalkylalkylrest, worin die aliphatische Kette 1 bis 4 Kohlenstoffatome enthält, ein Halogenatom, eine Nitrogruppe, eine Amingruppe, eine Aldehydgruppe, eine Nitrilgruppe darstellen,

– n eine Zahl von 0 bis 5 ist,

durch Reaktion mit einem Verätherungsmittel, ausgewählt aus der Gruppe bestehend aus den Alkylcarboxylaten, dem geradkettigen oder verzweigten Alkylrest mit 1 bis 6 Kohlenstoffatomen, den Alkenylcarboxylaten, dem geradkettigen oder verzweigten Alkenylrest mit 3 bis 6 Kohlenstoffatomen und den Verbindungen, die solche Carboxylate zu bilden vermögen, dadurch gekennzeichnet, dass man in Gegenwart wirksamer Mengen eines Carbonsäuresalzes, ausgewählt aus den Carboxylaten eines Alkalimetalls, von Ammonium oder eines Erdalkalimetalls, und eines Metallhalogenids, ausgewählt aus den Jodiden eines Alkalimetalls, den Jodiden eines Erdalkalimetalls, oder von Ammoniumjodid arbeitet.

2. Verfahren nach Anspruch 1, dadurch gekennzeichnet, dass es angewendet wird auf eine Verbindung der Formel (I), in welcher:

– der Rest Ar Benzol, Naphtalin, Anthracen oder Phenanthren entspricht,

– die Substituenten R unabhängig voneinander eine Hydroxylgruppe, einen geradkettigen oder verzweigten Alkylrest mit 1 bis 4 Kohlenstoffatomen, einen geradkettigen oder verzweigten Alkenylrest mit 2 bis 4 Kohlenstoffatomen, einen Phenylrest, einen Cyclohexylrest, einen Phenylalkylrest, in welchem die aliphatische Kette 1 bis 3 Kohlenstoffatome enthält, einen Cyclohexylalkylrest, in welchem die aliphatische Kette 1 bis 3 Kohlenstoffatome enthält, ein Chloratom, ein Bromatom oder eine Nitrogruppe darstellen,

– n eine ganze Zahl von 0 bis 3 ist.

3. Verfahren nach einem der Ansprüche 1 oder 2, dadurch gekennzeichnet, dass es angewendet wird auf Phenol, 1-Naphthol, 2-Naphthol, 2-Methylphenol, 3-Methylphenol, 4-Methylphenol, Monochlorphenole, Dichlorphenole, Monoäthylphenole, Mononitrophenole, Pyrokatechol, Resorcinol, Hydrochinon, 1,2-Dihydroxynaphthalin, 1,3-Dihydroxynaphthalin, 1,4-Dihydroxynaphthalin, 1,5-Dihydroxynaphtalin, 1,6-Dihydroxynaphthalin, 1,7-Dihydroxynaphthalin, 1,8-Dihydroxynaphthalin, 2,3-Dihydroxynaphthalin, 2,6-Dihydroxynaphthalin, 2,7-Dihydroxynaphthalin.

4. Verfahren nach einem der Ansprüche 1 bis 3, dadurch gekennzeichnet, dass das Verätherungsmittel ausgewählt wird aus den Estern:

– von gesättigten oder ungesättigten aliphatischen Carbonsäuren, die monofunktionell sind und insbesondere 2 bis 18 Kohlenstoffatome enthalten, oder polyfunktionell sind und insbesondere 3 bis 18 Kohlenstoffatome enthalten,

– von mono- oder polyfunktionellen aromatischen Säuren,

– von mono- oder polyfunktionellen arylaliphatischen Säuren, oder

– von mono- oder polyfunktionellen cycloaliphatischen Säuren mit Alkoholen wie Methanol, Äthanol, n-Propanol, Isopropanol, 1-Butanol, 2-Butanol, tert.-Butanol, 2-Propen-1-ol, 2-Methyl-2-propen-1-ol, 2-Buten-1-ol, 3-Buten-1-ol, 3-Buten-2-ol.

5. Verfahren nach einem der Ansprüche 1 bis 3, dadurch gekennzeichnet, dass das Verätherungsmittel besteht aus einer Mischung:

– von einem Alkohol wie Methanol, Äthanol, n-Propanol, Isopropanol, 1-Butanol, 2-Butanol, tert.-Butanol, 2-Propen-1-ol, 2-Methyl-2-propen-1-ol, 2-Buten-1-ol, 3-Buten-1-ol, 3-Buten-2-ol, und

– einer Carbonsäure wie z.B.:

– einer gesättigten oder ungesättigten aliphatischen Carbonsäure, die monofunktionell ist und insbesondere 2 bis 18 Kohlenstoffatome enthält, oder polyfunktionell ist und insbesondere 3 bis 18 Kohlenstoffatome enthält,

– einer mono- oder polyfunktionellen aromatischen Säure,

– einer mono- oder polyfunktionellen arylaliphatischen Säure, oder

– einer mono- oder polyfunktionellen cycloaliphatischen Säure.

6. Verfahren nach Anspruch 5, dadurch gekennzeichnet, dass das molare Verhältnis Alkohol/Carbonsäure zwischen 0,02 und 50, vorzugsweise zwischen 0,1 und 40 liegt.

7. Verfahren nach einem der Ansprüche 4 bis 6, dadurch gekennzeichnet, dass die einen Bestandteil des Verätherungsmittels bildende Carbonsäure eine gesättigte aliphatische mono- oder difunktionelle Säure mit 2 bis 6 Kohlenstoffatomen, Benzoesäure oder eine der Ortho-, Meta- und Terephthalsäuren ist.

8. Verfahren nach einem der Ansprüche 1 bis 7, dadurch gekennzeichnet, dass das verwendete Carbonsäuresalz ein Alkalimetallcarboxylat ist.

9. Verfahren nach einem der Ansprüche 1 bis 8, dadurch gekennzeichnet, dass das verwendete Carbonsäuresalz ein Salz einer gesättigten, aliphatischen mono- oder difunktionellen Säure mit 2 bis

6 Kohlenstoffatomen, von Benzoesäure oder einer der Ortho-, Meta- und Terephthalsäuren ist.

10. Verfahren nach einem der Ansprüche 1 bis 9, dadurch gekennzeichnet, dass das Gewichtsverhältnis Carbonsäuresalz/Phenolverbindung 0,01 bis 50 und vorzugsweise 0,05 bis 20 beträgt.

11. Verfahren nach einem der Ansprüche 1 bis 10, dadurch gekennzeichnet, dass das Metallhalogenid Natrium- oder Kaliumjodid ist.

12. Verfahren nach einem der Ansprüche 1 bis 11, dadurch gekennzeichnet, dass das Gewichtsverhältnis Metallhalogenid/Phenolverbindung zwischen 0,01 und 50, vorzugsweise zwischen 0,05 und 20 liegt.

13. Verfahren nach einem der Ansprüche 1 bis 12, dadurch gekennzeichnet, dass man in Gegenwart von Wasser arbeitet.

14. Verfahren nach Anspruch 13, dadurch gekennzeichnet, dass das Wasser 1 bis 95 Vol.-% des flüssigen Lösungsmittelmilieus, und vorzugsweise 20 bis 80%, ausmacht.

15. Verfahren nach einem der Ansprüche 1 bis 3 und 5 und 6, dadurch gekennzeichnet, dass man eine Carbondisäure verwendet, deren eine Säurefunktion frei und deren andere versalzt ist.

16. Verfahren nach einem der Ansprüche 1 bis 15, dadurch gekennzeichnet,dass man bei einer Temperatur im Bereich zwischen 150 und 350° C, vorzugsweise zwischen 200 und 300° C, arbeitet.

17. Verfahren nach einem der Ansprüche 1 bis 16, dadurch gekennzeichnet, dass man ein molares Verhältnis von einen Bestandteil des Verätherungsmittels bildendem Ester oder Alkohol/Phenolverbindung gleich oder grösser 0,3 und vorzugsweise gleich oder grösser 0,5 verwendet.

18. Verfahren nach einem der Ansprüche 1 bis 17, dadurch gekennzeichnet, dass die sich in der fertigen Reaktionsmasse befindenden, gebildeten Äther mit Cyclohexan extrahiert werden.

## Claims

1. A process for the etherification of a phenolic hydroxyl group of a compound having the structural Formula (I):

$$HO - Ar - (R)_n (I)$$

wherein

– Ar is an aryl radical comprising a benzene ring, or a plurality of ortho-fused or ortho- and peri-fused benzene rings,

– the substituents R, which are identical or different, represent a hydroxyl group, a linear or branched chain alkyl radical having 1 to 6 carbon atoms, a linear or branched chain alkenyl radical having 2 to 6 carbon atoms, a phenyl radical, a cycloalkyl radical, a phenylalkyl radical in which the aliphatic chain contains 1 to 4 carbon atoms, a cycloalkyl-alkyl radical in which the aliphatic chain contains 1 to 4 carbon atoms, a halogen atom, a nitro group, an amine group, an aldehyde group or a nitrile group, and

– n is a number ranging from 0 to 5,

by reaction with an etherification agent selected from the group consisting of alkyl carboxylates, the linear or branched chain alkyl radical having 1 to 6 carbon atoms, alkenyl carboxylates, the linear or branched chain alkenyl radical having 3 to 6 carbon atoms, and compounds capable of forming these carboxylates, and such reaction being characterized in that it is carried out in the presence of catalytically effective amounts of a salt of a carboxylic acid selected among an alkali metal carboxylate, ammonium carboxylate and an alkaline earth metal carboxylate and of a metallic halide selected among an alkali metal iodide, ammonium iodide and an alkaline earth metal iodide.

2. The process as defined by Claim 1, characterized in that it is applied to a compound of the Formula (I), wherein

– Ar is benzene, naphthalene, anthracene or phenanthrene,

– the substituents R, which are identical or different, represent a hydroxyl group, a linear or branched chain alkyl radical having 1 to 4 carbon atoms, a linear or branched chain alkenyl radical having 2 to 4 carbon atoms, a phenyl radical, a cyclohexyl radical, a phenylalkyl radical in which the aliphatic chain contains 1 to 3 carbon atoms, a cyclohexyl-alkyl radical in which the aliphatic chain contains 1 to 3 carbon atoms, a chlorine atom, a bromine atom or a nitro group, and

– n is an integer ranging from 0 to 3.

3. The process as defined by Claim 1 or 2, the compound (I) being phenol, 1-naphthol, 2-naphthol, 2-methylphenol, 3-methylphenol, 4-methylphenol, a monochlorophenol, a dichlorophenol, a monoethylphenol, a mononitrophenol, pyrocatechol, resorcinol, hydroquinone, 1,2-dihydroxynaphthalene, 1,3-dihydroxynaphthalene, 1,4-dihydroxynaphthalene, 1,5-dihydroxynaphthalene, 1,6-dihydroxynaphthalene, 1,7-dihydroxynaphthalene, 1,8-dihydroxynaphthalene, 2,3-dihydroxynaphthalene, 2,6-dihydroxynaphthalene or 2,7-dihydroxynaphthalene.

4. The process as defined by Claims 1 to 3, characterized in that the etherification agent is selected from the ester compounds of:

– monofunctional saturated or unsaturated aliphatic carboxylic acids containing 2 to 18 carbon atoms or polyfunctional saturated or unsaturated aliphatic carboxylic acids containing 3 to 18 carbon atoms,

– monofunctional or polyfunctional aromatic acids,

– monofunctional or polyfunctional arylaliphatic acids, or

– monofunctional or polyfunctional cycloaliphatic acids,

with alcohols such as methanol, ethanol, n-propanol, isopropanol, butan-1 ol, butan-2 ol, tert.-butanol, prop-2-en-1-ol, 2-methylprop-2-en-1-ol, but-2-en-1-ol, but-3-en-1-ol or but-3-en-2-ol.

5. The process as defined by Claims 1 to 3, the etherification agent being a carboxylate-forming admixture of an alcohol such as methanol, ethanol,

n-propanol, isopropanol, butan-1-ol, butan-2-ol, tert.-butanol, prop-2-en-1-ol, 2-methylprop-2-en-1-ol, but-2-en-1-ol, but-3-en-1-ol or but-3-en-2-ol, and of a carboxylic acid such as a monofunctional saturated or unsaturated aliphatic carboxylic acid containing 2 to 18 carbon atoms, a polyfunctional saturated or unsaturated aliphatic carboxylic acid containing 3 to 18 carbon atoms, a monofunctional or polyfunctional aromatic acid, a monofunctional or polyfunctional arylaliphatic acid, or a monofunctional or polyfunctional cycloaliphatic acid.

6. The process as defined by Claim 5, wherein the molar ratio alcohol/carboxylic acid ranges from 0.02 to 50 and preferably from 0.1 to 40.

7. The process as defined by Claims 4 to 6, the carboxylic acid of the etherification agent being a monofunctional or difunctional satured aliphatic acid having from 2 to 6 carbon atoms, benzoic acid or ortho-phthalic, meta-phthalic or terephthalic acid.

8. The process as defined by Claims 1 to 7, the salt of a carboxylic acid used in it being an alkali metal carboxylate.

9. The process as defined by Claims 1 to 8, wherein the carboxylic acid salt catalyst is a salt of monofunctional or difunctional saturated aliphatic acid having 2 to 6 carbon atoms, of benzoic acid or of ortho-phthalic, meta-phthalic or terephthalic acid.

10. The process as defined by Claims 1 to 9, wherein the weight ratio carboxylic acid salt/phenolic compound (I) ranges from 0.01 to 50 and preferably from 0.05 to 20.

11. The process as defined by Claims 1 to 10, wherein the metallic halide is sodium iodide or potassium iodide.

12. The process as defined by Claims 1 to 11 wherein the weight ratio metallic halide/phenolic compound (I) ranges from 0.01 to 50 and preferably from 0.05 to 20.

13. The process as defined by Claims 1 to 12, wherein the etherification reaction is carried out in the presence of water.

14. The process as defined by Claim 13, wherein the water constitutes from 1 to 95% by volume of the liquid reaction mixture and preferably from 20 to 80%.

15. The process as defined by Claims 1 to 3, 5 and 6, wherein the reaction mixture comprises a dicarboxylic acid, one acid function of which is free and the other is salified.

16. The process as defined by Claims 1 to 15, the etherification reaction being carried out at a temperature ranging from 150 to 350° C, preferably from 200 to 300° C.

17. The process as defined by Claims 1 to 16, wherein the molar ratio of the ester or alcohol comprised in the etherification agent to the phenolic compound (I) is at least 0.3 and preferably at least 0.5.

18. The process as defined by Claims 1 to 17, wherein the obtained ethers being in the final reaction mixture are extracted by cyclohexan.